# EUROPEAN PATENT APPLICATION

(11) **EP 2 568 288 A1**
(43) Date of publication of application: **13.03.2013**
(21) Application number: 11186491.4
(22) Date of filing: 25.10.2011
(51) Int. Cl.: G01N 33/569, B01L 3/00

(54) **Means and methods for staining acid-fast cells**

(30) Priority: 08.09.2011 US 532133 P
(71) Applicant: Koninklijke Philips Electronics N.V., 5621 BA Eindhoven (NL)
(72) Inventor: The designation of the inventor has not yet been filed
(74) Representative: Jungen, Carmen

(57) **Abstract**

The present invention relates to a method for specifically staining an acid-fast cell, in particular a Mycobacterium species, comprising the steps of: (a) contacting an acid-fast cell derived from a sample with an oil solution comprising an oil-soluble dye; and (b) mixing the acid-fast cell of step (a) with a lysis solution in which said dye is less soluble than in said oil solution, wherein said mixing causes an enrichment of said acid-fast cell in said oil solution and concomitantly an accumulation of said dye, in the interior of the acid-fast cell. The present invention also relates to the use of an oil solution comprising an oil-soluble dye and a lysis solution, in which said dye is less soluble than in said oil solution, for specifically staining an acid-fast cell, a kit of parts for specifically staining an acid-fast cell comprising an oil solution, an oil-soluble dye, and a lysis solution in which said dye is less soluble than in said oil solution, as well as sample collector suitable for processing and staining an acid-fast cell.

## Description

### FIELD OF THE INVENTION

The present invention relates to a method for specifically staining an acid-fast cell, in particular a Mycobacterium species, comprising the steps of: (a) contacting an acid-fast cell derived from a sample with an oil solution comprising an oil-soluble dye; and (b) mixing the acid-fast cell of step (a) with a lysis solution in which said dye is less soluble than in said oil solution, wherein said mixing causes an enrichment of said acid-fast cell in said oil solution and concomitantly an accumulation of said dye, in the interior of the acid-fast cell. The present invention also relates to the use of an oil solution comprising an oil-soluble dye and a lysis solution, in which said dye is less soluble than in said oil solution, for specifically staining an acid-fast cell, a kit of parts for specifically staining an acid-fast cell comprising an oil solution, an oil-soluble dye, and a lysis solution in which said dye is less soluble than in said oil solution, as well as sample collector suitable for processing and staining an acid-fast cell.

### BACKGROUND OF THE INVENTION

Tuberculosis is a contagious disease which if untreated often leads to death. It is caused by various mycobacteria, most prominently by *Mycobacterium tuberculosis.* Tuberculosis is most known for affecting the lungs, however, it can also affect other organs. It is transmitted through the air when patients cough, sneeze, or spit. If untreated, a patient with active disease on average infects 10-15 other people per year. Most tuberculosis infections lead to an asymptomatic, latent infection. 10% of these latent infections eventually progresses to active disease, which, if left untreated, kills more than 50% of its victims. In 2009 9.4 Million new tuberculosis cases occurred and 1.7 Million people died from tuberculosis worldwide. It is estimated that about one third of the world's population is infected. In 1993, the World Health Organization declared tuberculosis a global emergency, the only disease that has been declared so up until today.

Tuberculosis is a disease of poverty that disproportionately affects poor countries and marginalized populations. 95% of all tuberculosis cases and 98% of all tuberculosis deaths occur in developing countries, with most deaths occurring in sub-Saharan Africa and Asia.

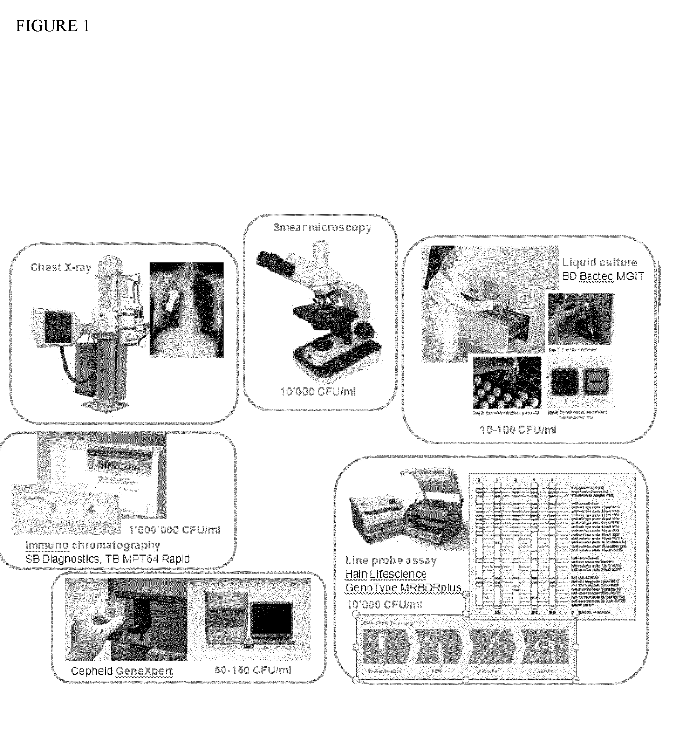

In the recent years previously used bacterial diagnosis techniques have been improved and several new diagnosis techniques have been developed including chest X-ray approaches, smear microscopy, liquid culture tests (which is considered the gold standard in the field), line probe assays (i.e. the hybridization of nucleic acids), nucleic acid amplification tests (e.g. based on Cepheid GeneExpert), and immune-chromatography assays (see, for example, Figure 1 which provides a graphical overview). However, many of these will in the near future only be available for markets of developed countries or regions due to limited financial and scientific or medical resources in high burden regions.

In these under-developed zones typically sputum smear microscopy methods with limited sensitivity are used, which are still based on a methodology developed by Franz Ziehl and Friedrich Neelsen over 100 years ago.

The underlying principle of specifically detecting mycobacteria is based on the acid-fastness of this bacterial group. Acid-fastness is a physical property of some bacteria referring to their resistance to decolorization by acids during staining procedures. This allows for specific staining of mycobacteria in a sample, e.g. a sputum sample, while other bacteria residing in the respiratory tract may be counterstained with another dye. For sputum smear microscopy three sputum samples need to be taken, the sputum needs to be smeared out on microscopic slides, fixed and stained in a series of steps, called the acid fast or Ziehl-Neelsen staining. This method was originally developed in 1882 by Ehrlich, who described the acid fast properties of the organism. He was unable to remove the stain from mycobacteria with dilute acids after staining with a fuchsin-aniline oil mixture. Modifications to that method were made by Ziehl in 1882 and 1883, who first used basic fuchsin with carbolic acid (phenol). In 1883 Neelsen used the same staining solution as Ziehl but destained tissue elements with 25% sulphuric acid. Substitutes for basic fuchasin in the original method have included such dyes as Night blue, Victoria Blue R, triphenyl and diphenyl naphthylamine dyes with pararosaniline and rosaniline, and new fuchsin. Also the phenol was replaced by less toxic substances such as LOC High Suds (Ellis and Zabrowarny, 1993, J Clin Pathol, 46, 559-560).

Subsequent to the staining the sample needs to be analysed by a trained person. To be able to diagnose correctly, at least 300 microscopic fields of view need to be observed for standard brightfield microscopy or at least 40 fields with fluorescent microscopy. This observation takes a skilled technician approximately a quarter of an hour, so only a limited amount of samples can be diagnosed each day (estimations are approximately 25). Also, the preparation of the slides itself contains many steps and is too error-prone to be used by little-trained personnel. Furthermore, the sensitivity of the Ziehl-Nielsen methods is limited to approximately 45%.

In consequence, there is a need for the development of an improved and simplified staining methodology for acid-fast cells, in particular acid-fast bacteria, which provides an increased sensitivity, is automatable and can be used in regions without complex and developed diagnostic or scientific infrastructure.

### OBJECTS AND SUMMARY OF THE INVENTION

The present invention addresses these needs and provides means and methods for the staining of acid-fast cells. The above objective is in particular accomplished by a method for specifically staining an acid-fast cell, comprising the steps of: (a) contacting an acid-fast cell derived from a sample with an oil solution comprising an oil-soluble dye; and (b) mixing the acid-fast cell of step (a) with a lysis solution in which said dye is less soluble than in said oil solution, wherein said mixing causes an enrichment of said acid-fast cell in said oil solution and concomitantly an accumulation of said dye in the interior of the acid-fast cell. This newly developed methodology is based on the properties of acid-fast cells, in particular mycobacteria, which make them acid-fast, i.e. their unique cell wall which comprises very hydrophobic entities such as mycolic acids. A key element is the up concentration of the acid fast cells, e. g. mycobacteria in oily substances while other cells or bacteria, e.g. respiratory tract bacteria, stay in a sample such as sputum, which was already described by Lange and Nitsche, 1909, Deutsche Medizinische Wochenschrift 35:435-436. Importantly, as the concentration step is specific for acid-fast cells, in particular for mycobacteria, the staining method does not need to be specific anymore, i.e. only acid-fast cells are significantly enriched in the oily substance. However, the staining step has to be carried out such that an accumulation of the dye in the interior of acid-fast cells in an oily phase becomes possible. Such an accumulation, e.g. in bacteria, requires a dye which has a low solubility in the aqueous phase, a higher solubility in the oily substance and an even higher solubility in the cytoplasm of the cell. The staining has to take place in cells that are dissolved in oil.

Since after the enrichment only acid-fast cells will be present in the oily substance, all cells, e. g. bacteria, which are stained in this oily substance are acid-fast cells. This essentially avoids the traditional acid-fast or Ziehl-Neelsen staining performed by Lange and Nitsche, as well as others.

The presently provided approach thus tremendously simplifies the staining procedure taking away the necessity of the multiple steps of the acid-fast staining procedure. The method is furthermore so simple and based on the use of so few reagents, that it is well suited for the use in high burden regions.

Due to its simplicity it can also be used in more complex medical environment, e.g. in combination with molecular detection or diagnostic techniques. Thus, in a preferred embodiment the method as described above additionally comprises as step (c) a molecular diagnostic step. In a particularly preferred embodiment, a detection of an acid-fast cell may be carried out via a molecular detection technique.

In a further preferred embodiment of the present invention said molecular diagnostic step comprises the extraction of water soluble contents from lysed acid-fast cell into an aqueous solution followed by the performance of a molecular detection technique in said aqueous solution. In a particularly preferred embodiment the diagnostic step comprises the extraction of DNA or proteins into an aqueous solution, followed by the performance of a molecular detection technique in said aqueous solution.

In another preferred embodiment of the present invention the sample is a stool, whole blood, serum, plasma, tears, saliva, nasal fluid, sputum, ear fluid, genital fluid, breast fluid, milk, colostrum, placental fluid, amniotic fluid, perspirate, synovial fluid, ascites fluid, cerebrospinal fluid, bile, gastric fluid, aqueous humor, vitreous humor, gastrointestinal fluid, exudate, transudate, pleural fluid, pericardial fluid, semen, upper airway fluid, peritoneal fluid, fluid harvested from a site of an immune response, fluid harvested from a pooled collection site, bronchial lavage, or urine sample, a soil or environmental water sample, a oil waste sample, or a biopsy material sample.

In a further aspect the present invention relates to the use of an oil solution comprising an oil-soluble dye and a lysis solution in which said dye is less soluble than in said oil solution for specifically staining an acid-fast cell.

In yet another aspect the present invention relates to a kit of parts for specifically staining an acid-fast cell, wherein said kit comprises an oil solution comprising an oil-soluble dye, and a lysis solution in which said dye is less soluble than in said oil solution.

In another aspect the present invention relates to a sample collector suitable for processing and staining an acid-fast cell, comprising a cap element containing a lysis solution, wherein said cap element comprises a predetermined breaking point allowing the lysis solution to flow out of said cap element, and a receptacle containing an oil solution comprising an oil-soluble dye, wherein said dye is less soluble in said lysis solution than in said oil solution.

In a preferred embodiment of the herein above mentioned method, use, kit of parts or sample collector of the present invention, said lysis solution is an alkaline lysis solution having a pH of about 10.5 or higher and said dye is an alkaline dye. In a particularly preferred embodiment said lysis solution is an alkaline lysis solution having a pH of about 12 or higher and said dye is an alkaline dye.

In another preferred embodiment of the herein above mentioned method, use, kit of parts or sample collector of the present invention, said lysis solution is an acid lysis solution having a pH of about 4 or lower and said dye is an acid dye. In a particularly preferred embodiment said lysis solution is an acid lysis solution having a pH of about 3.5 or lower and said dye is an acid dye.

In yet another preferred embodiment of the herein above mentioned method, use, kit of parts or sample collector of the present invention, said lysis solution is a redox lysis solution comprising a lysis solution with a lower oxidative power than the cytoplasm of an acid fast bacterium and said dye is a basic redox dye which is in an uncharged, oil soluble state.

In a further preferred embodiment of the present invention said alkaline dye as mentioned herein above is Acridine orange, 1,4-phenylenediamine, Bismarck brown, quinacrine, N-4-amino-2,5 diethoxy phenyl benzamide, Nigrosin, Nile Blue A, Sudan Blue II, Sudan Black B or Solvent Green 3. In a particularly preferred embodiment said alkaline dye is Acridine orange.

In a further preferred embodiment of the present invention said acid dye as mentioned herein above is 2-naphthol, 4-chloro-1-naphthol, oil red o, rose Bengal, bromophenol blue, carminic acid, phloxine b, picric acid, eosin (solvent red 45), erythrosine, fluoresceine 5 (6) isothiocyanate, hematoxyline, hesperetin, morin, Resorufin sodium salt, Sudan IV or Sudan Orange G

In yet another preferred embodiment of the present invention said basic redox dye as mentioned herein above is Auramine, azure a, azure b, azure II, brilliant green, brilliant cresyl blue, fuchsin, ethyl violet, toluidine blue, anus green B, cresyl violet, methylene blue, methylene green, malachite green, methyl violet, New Methylene Blue, Neutral Red, Phenosafranine, Pinacyanol iodide, Rhodamine B, or Safranin O.

In another preferred embodiment of the present invention said oil as mentioned herein above has a partition coefficient of more than 10⁴ in octanol/water. In a particularly preferred embodiment of the present invention said oil has a partition coefficient of between 10⁵ and 10⁸ in octanol/water.

In a further preferred embodiment of the present invention said oil is an oil from an animal source, such as fish oil, lard oil, cod liver, or butter; an oil from a vegetable source, such as olive oil, sunflower oil, peanut oil, soybean oil, or corn oil; a mineral oil, such as paraffin oil, naphtenic oil, or aromatic oil; or a silicon oil.

In yet another preferred embodiment of the present invention said acid-fast cell as mentioned herein above is an acid-fast bacterium. In a further preferred embodiment said acid-fast bacterium is a mycobacterium, preferably a bacterium of the Mycobacterium tuberculosis complex, a bacterium of the Mycobacterium avium complex, a bacterium of the group of mycolactone-producing mycobacteria, or a leprosy causing mycobacterium; or a bacterium of the genus Nocardia, preferably Nocardia asteroids; or a bacterium of the genus Rhodococcus, preferably Rhodococcus equi.

In a particularly preferred embodiment of the present invention said mycobacterium is Mycobacterium tuberculosis, Mycobacterium bovis, Mycobacterium africanum, Mycobacterium canetti, Mycobacterium microti, Mycobacterium avium, Mycobacterium ulcerans, Mycobacterium leprae, or Mycobacterium lepromatosis. Most preferably, the mycobacterium is Mycobacterium tuberculosis.

### BRIEF DESCRIPTION OF THE DRAWINGS

Fig. 1 depicts various diagnostic methods currently available; from top left, clockwise: chest X-ray, smear microscopy, liquid culture, line probe assay (i.e. hybridization of nucleic acids), nucleic acid amplification tests (e.g. Cepheid GeneExpert), and immuno chromatography.

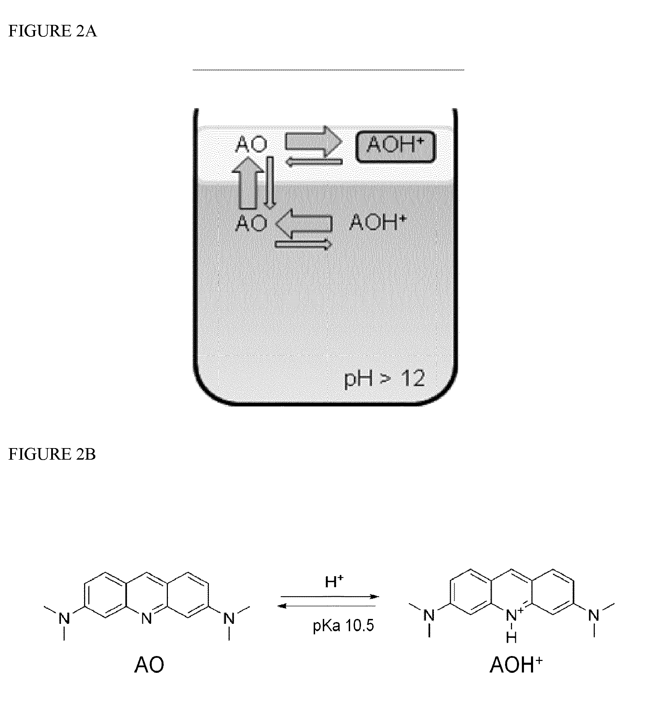

Fig. 2 illustrates the principle of staining mycobacteria in mineral oil containing a hydrophilic/hydrophobic dye and shows that staining of mycobacteria in oil works. It also shows that another advantage of having the cells in oil is that they have much less tendency to clump together compared to mycobacteria in aqueous solutions. In Fig. 2A chemical equilibria of dye molecules are shown. In particular, the described dye molecules can be protonated/deprotonated depending on their surroundings. As an example Acridine orange base is presented in Fig. 2B. Acridine orange (AO) has a pKa of 10.5, meaning that at pH higher than 10.5 the majority of the Acridine orange molecules is deprotonated. Therefore they lose their charge and become hydrophobic and will thus enter the oil phase. The liquefied sputum has a pH > 12 as during the described procedure it is decontaminated with NaOH (>0.5% w/v). Once the Acridine orange molecules are in the oil phase they can easily equilibrate with the very hydrophobic cell wall of the mycobacteria. If an Acridine orange molecule reaches the other side of the cell wall (=inside of the cell), the molecule becomes once more protonated as the pH at the inside of the cell is <<10.5. Thus it becomes charged and cannot penetrate the cell wall anymore, which leads to an accumulation of the positively charged Acridine orange molecules inside the mycobacteria. A positive charge of an Acridine orange molecule also renders it much more fluorescent than the deprotonated form. This leads to brightly fluorescent mycobacteria in front of a much less fluorescent background.

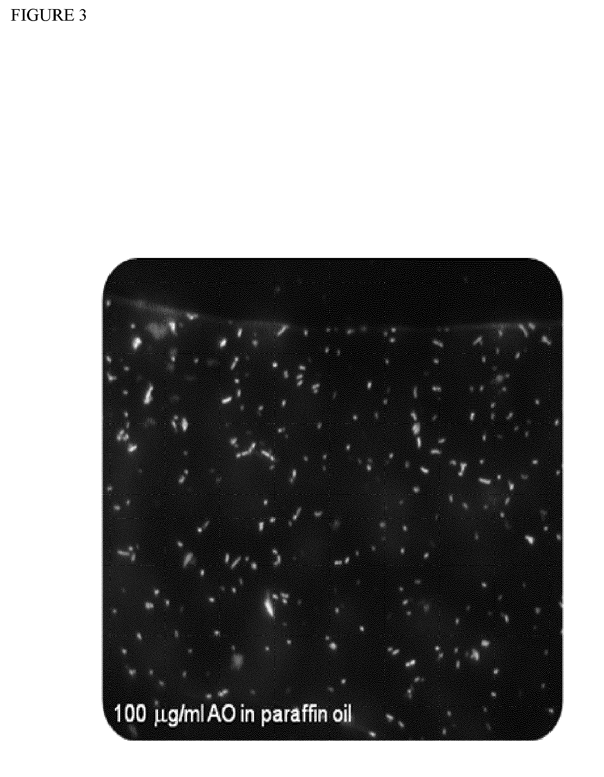

Fig. 3 shows mycobacteria that were directly dissolved in paraffin oil containing 100 µg/ml Acridine orange. The picture was taken with a 100 X oil immersion objective.
Figs. 4A to 4D show different steps of staining of mycobacteria in oil after extraction from aqueous solution and inactivation as explained in Example 2. The picture was taken with a 100X oil immersion objective.
Fig. 5 shows the different colors of stained mycobacteria, which are dependent on dye concentration. Mycobacteria were resuspended in oil containing varying concentrations of Acridine orange base. The ratio of red versus green bacteria was estimated visually after taking microscopic pictures.

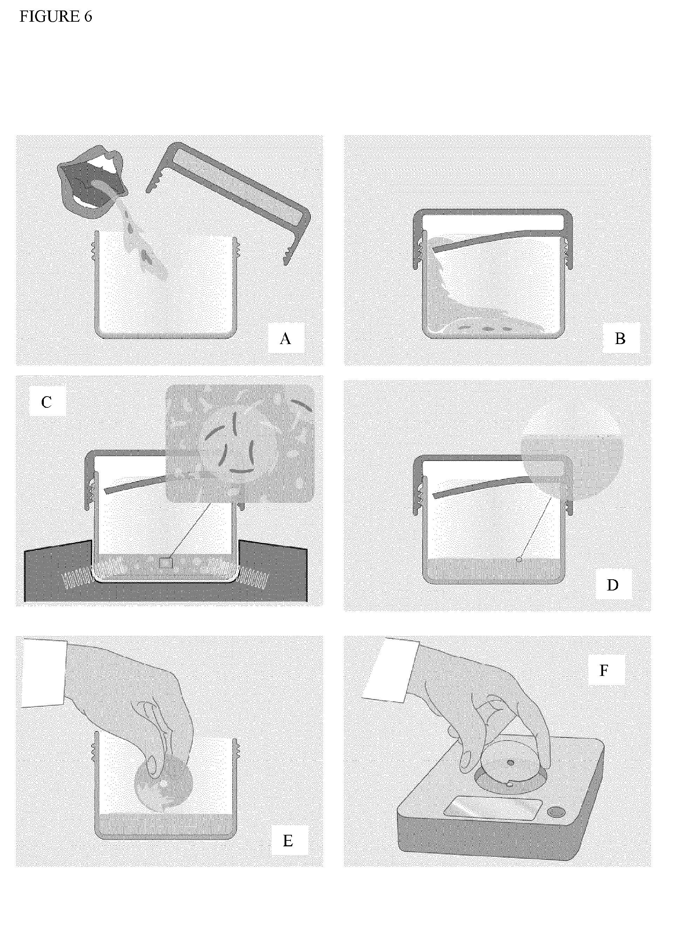

Fig. 6 illustrates a sample collector suitable for processing and staining an acid-fast cell and its use. In Fig. 6A a sample collector is depicted which comprises in the bottom of a receptacle an oil solution comprising an oil-soluble dye. Furthermore, the figure shows a cap element containing a lysis solution, wherein the cap element comprises a predetermined breaking point. A patient provides a sputum sample by spitting directly into the sample collector. In Fig. 6B the predetermined breaking point in the cap element is broken and the lysis solution flows out of the cap element into the receptacle section, joining the sample, the oil solution and the oil-soluble dye. Fig. 6C shows a mixture of the components, allowing for an enrichment of the acid-fast cells in the oil solution. The mixture process may be assisted by the application of external heat or a mild sonication step In Fig. 6D a separation of the phases is shown, with acid-fast bacteria comprising the oil-soluble dye in the upper oily phase. Fig. 6E shows how the accordingly enriched cells can be derived from the sample collector on slide compatible with a hand held diagnostic device. Finally, in Fig. 6F the slide may be visually inspected and analysed in a handheld or small bench-top diagnostic device.

### DETAILED DESCRIPTION OF THE EMBODIMENTS

The present invention relates to a method for specifically staining an acid-fast cell.

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As has been set out above, the present invention concerns in one aspect a method for specifically staining an acid-fast cell, comprising the steps of: (a) contacting an acid-fast cell derived from a sample with an oil solution comprising an oil-soluble dye; and (b) mixing the acid-fast cell of step (a) with a lysis solution in which said dye is less soluble than in said oil solution, wherein said mixing causes an enrichment of said acid-fast cell in said oil solution and concomitantly an accumulation of said dye, in the interior of the acid-fast cell.

The term "specifically staining" as used herein refers to the claimed methods' property of ending up with stained acid-fast cells, whereas cells, e.g. bacteria, derived from other groups are not stained. The distinguishing element, i.e. the activity which allows to specifically detect acid-fast cells resides in the interplay between the contacting with the oil solution, whereby a specific enrichment of the acid-fast bacteria becomes possible, and the subsequent staining step. The staining methodology, although intimately linked to the oil-solution enrichment, may be unspecific or may stain also non acid-fast cells. By combining both steps, a specific staining of acid-fast cells becomes possible.

The term "acid-fast" or "acid-fast cell" as used herein refers to the physical property of a cell to resist decolorization by acids during traditional staining procedures. Typically, a high mycolic acid content of a cell wall may contribute to this behavior. Acid-fast cells may comprise several bacterial groups, in particular bacteria of the Mycobacterium complex, Nocardia species, Rhodococcus species, bacterial spores, e.g. endospores, as well as protozoal species such as Cryptosporidium, Isospora and Cyclospora.

The term "oil solution" as used herein refers to an oil composition which is liquid at room temperature and above. Alternatively, the oil solution may be comprised of matter being solid at room temperature, which becomes liquid upon an increase of temperature, e.g. to 30-40°C. In the liquid phase, the composition or oil solution essentially does not dissolve in water. For example, the oil solution in its liquid phase may, in some embodiments, be about 10,000 times, about 50,000 times, about 100,000 times, about 150,000 times, about 250,000 times, about 500,000 times, or about 750,000 times more soluble in oil, than in H₂O. In other, preferred embodiments, the oil may be about 1,000,000 times more soluble in oil, than in H₂O

In a particularly preferred embodiment the oil solution comprises, essentially consists of or consist of oil which has a partition coefficient of more than about 10⁴ in octanol/water, e.g. of about 10⁵ in octanol/water, about 10⁶ in octanol/water, about 10⁷ in octanol/water, about 10⁸ in octanol/water, about 10⁹ in octanol/water, or about 10¹⁰ in octanol/water, or any value in between the indicated values. In a further preferred embodiment said oil may have a partition coefficient of between 10⁵ and 10⁸ in octanol/water.

In further preferred embodiments of the invention the oil solution comprises, essentially consists of, or consists of oil from an animal source. Preferred examples of such animal oil are fish oil, lard oil, cod liver, or butter. In further preferred embodiments the oil solution comprises, essentially consists of, or consists of oil from a vegetable source. Preferred examples of such vegetable oil are olive oil, sunflower oil, peanut oil, soybean oil, or corn oil. In further preferred embodiments the oil solution comprises, essentially consists of, or consists of mineral oil. Preferred examples of such mineral oil are paraffin oil, naphtenic oil, or aromatic oil. In further preferred embodiments the oil solution comprises, essentially consists of, or consists of silicon oil. In yet another group of embodiments, the above mentioned oils may be present in combination, or be combined with further ingredients or substances

The term "oil-soluble dye" as used herein refers to a dye which is in at least one of its chemical forms of appearance essentially lipophilic. Preferably, an oil-soluble dye according to the present invention is a dye which is soluble in an oil solution as defined herein above or below. The solubility in the oil solution of the oil-soluble dye may vary or change, e.g. in dependence of the pH, e.g. a low or high pH, and/or the redox or oxidation state of the dye. For example, an oil-soluble dye may be lipophilic or hydrophobic in dependence of its pKa. Thus, in the presence of a pH higher than its pKa, e.g. at a pH of about 10 or higher, the dye may become deprotonated and, in consequence, lipophilic or hydrophobic. In this state, the dye may be "oil-soluble".

In a particularly preferred embodiment the oil-soluble dye may be an alkaline dye, being oil soluble at a pH of about 10.5 or higher, e.g. at a pH of 11, 11.5, preferably of about 12 or higher.

In a further particularly preferred embodiment the alkaline dye is Acridine orange, 1,4-phenylenediamine, Bismarck brown, quinacrine, N-4-amino-2,5 diethoxy phenyl benzamide, Nigrosin, Nile Blue A, Sudan Blue II, Sudan Black B or Solvent Green 3. Particularly preferred is Acridine orange. The present invention also envisages further, chemically similar alkaline dyes, which would be known to the person skilled in the art or can be derived from suitable literature sources such as Conn's biological stains, 2002, Garland Pub, 10th ed. The present invention also envisages chemically similar alkaline dyes which are not yet know or may be known to the skilled person in the future.

In other examples, an oil-soluble dye may be hydrophilic at neutral or high pH, but hydrophobic or lipophilic at low pH, e.g. at about pH 3 to 5.

In a particularly preferred embodiment the oil-soluble dye may accordingly be an acid dye, being oil soluble at a pH of about 4 or lower, preferably of about 3.5 or lower, e.g. at about 3.0, 2.5 or 2.0 etc.

In a further particularly preferred embodiment the acid dye may be 2-naphthol, 4-chloro-1-naphthol, oil red o, rose Bengal, bromophenol blue, carminic acid, phloxine b, picric acid, eosin (solvent red 45), erythrosine, fluoresceine 5 (6) isothiocyanate, hematoxyline, hesperetin, morin, Resorufin sodium salt, Sudan IV or Sudan Orange G. The present invention also envisages further, chemically similar acid dyes, which would be known to the person skilled in the art or can be derived from suitable literature sources such as Conn's biological stains, 2002, Garland Pub, 10th ed. The present invention also envisages chemically similar acid dyes which are not yet know or may be known to the skilled person in the future.

In yet another example, the oil solubility of the dye may be dependent on reduced or non-oxidized state of the molecule. Thus, in a non-oxidizing or reducing environment, the dye molecule may be oil soluble, whereas its oil-solubility may change or be reduced or vanish in an oxidizing or non-reducing environment.

In a particularly preferred embodiment the oil-soluble dye may accordingly be a basic redox dye. Particularly preferred examples of basic redox dye are Auramine, azure a, azure b, azure II, brilliant green, brilliant cresyl blue, fuchsin, ethyl violet, toluidine blue, j anus green B, cresyl violet, methylene blue, methylene green, malachite green, methyl violet, New Methylene Blue, Neutral Red, Phenosafranine, Pinacyanol iodide, Rhodamine B, and Safranin O. The present invention also envisages further, chemically similar basic redox dyes, which would be known to the person skilled in the art or can be derived from suitable literature sources such as Conn's biological stains, 2002, Garland Pub, 10th ed. The present invention also envisages chemically similar basic redox dyes which are not yet know or may be known to the skilled person in the future.

The amount of dye in the oil solution may vary in dependence on the chemical property of the dye. Further details are known to the skilled person or can be derived from product information sheets or suitable handbooks. For example, oil solution may comprise about 10 µg/ml, 50 µg/ml, 100 µg/ml, 200 µg/ml, 400 µg/ml, 500 µg/ml dye.

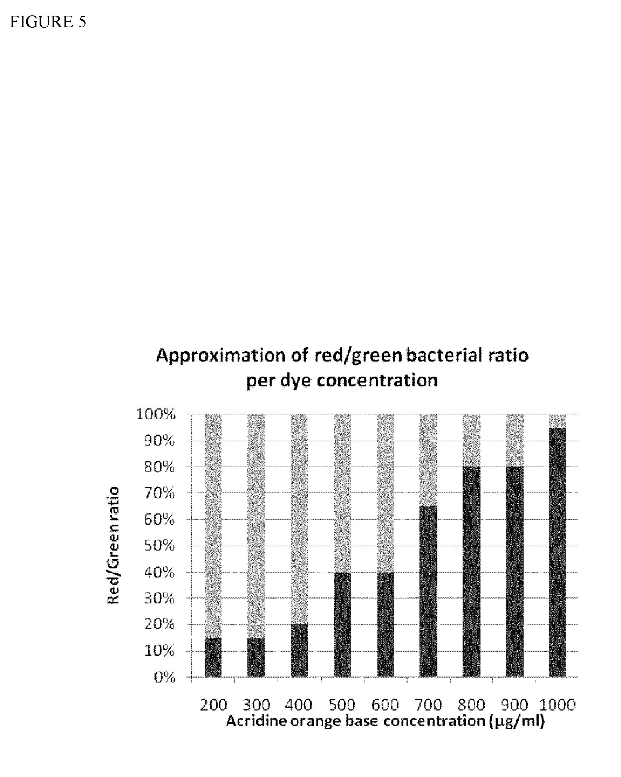

In specific embodiments of the present invention Acridine orange may be used as dye in concentrations of about 100 µg/ml to about 1500 µg/ml, preferably in a range of about 200 µg/ml to about 1000µg/ml in the oil solution, e.g. at a concentration of about 200 µg/ml, about 300 µg/ml, about 400 µg/ml, about 500 µg/ml, about 600 µg/ml, about 700 µg/ml, about 800 µg/ml, about 900 µg/ml, or about 1000 µg/ml in the oil solution. As has been found by the present inventors, the shifting of concentration of Acridine orange leads to a shifting of colors from green to red when increasing the concentration of Acridine orange form 200 µg/ml to 1000 µg/ml in the oil solution (see also Fig. 5). This color shifting may be used in accordance with the present invention for a fine tuning of the staining procedure in dependence of the sample type, sample origin and the present background color. For example, in case a strong green background is present in the sample, the concentration of Acridine orange may be increased towards or set at higher concentrations of about 700 to 1000 µg/ml such that the stained cells may become better distinguishable or visible due to their red staining. Similarly, in case a strong red background is present in the sample, the concentration of Acridine orange may be decreased towards or set at lower concentrations of about 200 to 400 µg/ml such that the stained cells may become better distinguishable or visible due to their green staining. The same principle may be employed for adjusting the staining of other dyes as described herein.

In a further embodiment, the dye may be selected in dependence of the sample type, sample origin and the background color. The present invention accordingly envisages a method comprising additional steps of checking the sample background, selecting the appropriate dye, e.g. the dye providing the highest contrast or easiest distinguishability, and optionally, a step of fine-tuning the concentration of dye in the oil solution in dependence of the sample type, sample origin and the background color.

In yet another embodiment of the present invention, the color shifting property of Acridine orange within the methodology of the present invention may be used for measuring the amount or concentration of acid-fast cells, e.g. acid fast bacteria, in a sample, For example, a dye, preferably Acridine orange may be employed in an initial high concentration of about 700 µg/ml to about 1000 µg/ml in the oil solution. In this concentration a red color staining may be expected at a predetermined concentration of cells. Should, however, the concentration of acid-fast cells, e.g. acid-fast bacteria, in the sample surpass the threshold, the dye may be diluted within more cells, leading to a lower concentration of dye within the cells and shifting the staining towards a green color staining. This approach may advantageously be accompanied by calibration and/or control staining. Furthermore, the method may be automated for a quick check on acid-fast cell, e.g. acid-fast bacteria, load of a patient, or of several samples, e.g. derived from the same patient, different patients, or different patient groups or populations.

In a particularly preferred embodiment of the present invention, the oil solution may comprise about 100µg/ml Acridine orange.

In further embodiments of the present invention, more than one dye, e.g. 2, 3, 4, or more dyes may be used in the oil solution, provided the dye is from the same functional group, i.e. an alkaline dye, an acid dye or a basic redox dye.

The term "oil solution comprising an oil-soluble dye" as used herein refers to an oil solution as defined herein above, which comprises an oil-soluble dye as defined herein above, i.e. a dye as defined herein above in the oil soluble state.

The term "lysis solution in which said dye is less soluble than in said oil solution" as used herein refers to an aqueous solution able to lyse and/or decontaminate a sample comprising a cell, in particular a bacterial cell, in which a dye according to the present invention is generally less prone to be soluble, than in an oil solution as defined herein above or below. The lysis solution may preferably be provided with a property corresponding to or complementing the properties of the dye allowing its solubility in the oil solution as defined herein above or below. The lysis solution may comprise in addition to the mentioned compounds further ingredients necessary for sterilizing the sample or for killing cells, in particular pathogenic cells.

For example, if the solubility in the oil solution of the oil-soluble dye depends on, or is given at, a high pH, a lysis solution according to the present invention to be employed with said dye, i.e. an alkaline dye as described above, may have such a high pH. In a preferred embodiment, the lysis solution may accordingly be an alkaline lysis solution, more preferably, the lysis solution may have a pH of about 10.5 or higher, even more preferably of about 12 or higher. This lysis solution is advantageously used with the above described alkaline dye in order to achieve the staining effect according to the present invention.

If, on the other hand, the solubility in the oil solution of the oil-soluble dye depends on, or is given at, a low pH, a lysis solution according to the present invention to be employed with said dye may have such a low pH. In a preferred embodiment, the lysis solution may accordingly be an acid lysis solution, more preferably the lysis solution may have a pH of about 4 or lower, even more preferably of about 3.5 or lower. This lysis solution is advantageously used with the above described acid dye in order to achieve the staining effect according to the present invention.

If, as a further example, the solubility in the oil solution of the oil-soluble dye depends on, or is given at a specific reduction or oxidation state of the molecule, a lysis solution according to the present invention to be employed with the dye may provide for such a reduction or oxidation state. In a preferred embodiment the lysis solution may accordingly be a redox lysis solution comprising a lysis solution with a lower oxidative power than the cytoplasm inside an acid fast cell. This lysis solution is advantageously used with the above described basic redox dye in order to achieve the staining effect according to the present invention.

Thus, by employing a lysis solution contributing to a hydrophobic or lipophilic character of the dye, the dye itself may be less soluble in said lysis solution than in said oil solution. Examples of such useful lysis solutions are a basic or strongly basic solution, for example a NaOH, or KOH solution, preferably a lysis solution comprising between about 0.5 to 10% NaOH; or an acid lysis solution or a strongly acid lysis solution such as an HCl solution, or a H₂SO₄ solution; or a non-oxidizing or reducing lysis solution, for example a solution comprising ascorbic acid, or a solution comprising ascorbic acid and a second compound such asan acid or base, e.g. HCl, H₂SO₄, or NaOH or KOH.

The pH or oxidation state may accordingly contribute to the electric charge of the dye molecule, or its protonation or deprotonation. Depending on the presence of an electric charge in the dye molecule, the dye molecule may be hydrophilic. Upon the deprotonation or vanishing of the electric charge, the dye molecule may become hydrophobic or lipophilic.

Preferred additional ingredients of the lysis solution are N-acetyl-cysteine, and/or one or more proteases.

In further specific embodiments of the present invention the oil-solubility of the dye may be observable in equilibrium states between an oil solution as defined herein, a dye molecule as defined herein and a lysis solution as defined herein. In these equilibria the dye may tend to stay in the oil solution.

The term "contacting" as used herein means resuspending an acid-fast cell or group of cells derived from a sample, or present in a sample, in an oil solution according to the present invention as defined herein. The contacting may be carried out directly with a sample, e.g. a liquid sample, or semi-liquid sample. Alternatively, the contacting may be carried out after preparation steps such as a cleansing of sample elements, resuspending sample elements, the separation of medium residuals from the sample, the addition of buffer solutions etc., and/or the performance of independent decontamination steps such as a treatment with ultraviolet radiation, or with microbizide solutions, or after a heating step. The contacting may be performed as a joining of solutions. It may also be accompanied by a mixing activity, e.g. a short vortexing or swinging of the solutions, e.g. for about 5 sec, 10 sec, 20 sec, 30 sec or longer or any value in between the indicated values. Alternatively or in addition, the contacting may be accompanied by, or assisted by the application of ultrasound waves, e.g. by using a suitable ultrasound generator. Ultrasound waves may be applied for about for about 5 sec, 10 sec, 20 sec, 30 sec or longer or any value in between the indicated values.

The term "mixing the acid-fast cell with a lysis solution" as used herein means that the acid-fast cells present in the above mentioned oil solution are subsequently mixed with a lysis solution as defined herein. The mixing may be carried out directly after the contacting of the cells with the oil solution as described herein. In further embodiments, the mixing may also be carried out after a certain period of time, e.g. after about 30 sec, 45 sec, 1 min, 2 min, 5 min, 10 min, 30 min, 60 min or longer or any value in between the indicated values. In further embodiments, the mixing and the contacting step as described above may be carried out at the same time, i.e. the cell or group of cells derived from a sample may be mixed with an oil solution as described herein, as well as with a lysis solution as described herein at the same time. In specific embodiments, the mixing steps may be carried out after an additional incubation or culturing steps in the oils solution, e.g. after about 10 h to 72 h. Corresponding culturing or growing conditions are preferably made dependent on the cells to be cultured and would be known to the person skilled in the art.

The mixing may be performed as vortexing or vigorous shaking of the solutions, e.g. for about 5 sec, 10 sec, 20 sec, 30 sec, 60 sec, 2 min, 5 min or longer or any value in between the indicated values. In further embodiments of the present invention, the mixing may be carried out by a sequence of mixing, i.e. vortexing or vigorous shaking steps followed by a period of resting, followed by a further mixing etc. For example, such a sequence may have the form of about 1 min mixing, followed by about 1-5 min resting, preferably 5 min resting, followed by about 1 min mixing, followed by about 1-5 min resting, preferably 5 min resting. This sequence of steps may be repeated once, two times, 2, 4, 5, 6, 7 or more times. The mixing and/or resting steps may further be modified individually, i.e. the mixing may be carried out for less than 1 min, e.g. 30 sec, or more than 1 min, e.g. 2 min, 3 min etc. Also the resting steps may be modified, e.g. a resting phase may take less than 1 min, e.g. 30 sec, or more than 5 min, or any value in between the indicated values.

As a result of the mixing the acid fast cells enrich in said oil solution. The term "enrich" or "enrichment" as used herein refers to the collection or accumulation of acid fast cells in the oil solution. The cells may accordingly switch or transfer from an aqueous sample solution to said oil solution upon the provision of said oil solution. Typically, acid-fast cells, in particular acid fast bacteria due to their specific cell wall structure, tend to solve or accumulate in an oil solution as described herein. Other cells, in particular other bacteria of the non-acid-fast group, do not tend to solve or accumulate in said oil solution. By providing the oil-solution and the corresponding contacting step, the present invention, thus allows for a specific separation of acid-fast and non-acid fast cells, in particular, of acid-fast bacteria and non-acid fast bacteria.

The result of the mixing procedure, i.e. the enrichment of acid fast cells, e.g. acid-fast bacteria in the oil solution may present itself, e.g. after a few moments of resting, e.g. after several seconds, 30 sec, 45 sec, 1 min, 2 min, 2.5 min, 3 min, 4 min, 5 min or longer of resting the mixture in situ, e.g. at room temperature. Alternatively or in addition, the enrichment may be enforced by the application of ultrasound waves, e.g. by using a suitable ultrasound generator. Ultrasound waves may be applied for about 5 sec, 10 sec, 20 sec, 30 sec or longer or any value in between the indicated values. In an alternative embodiment, the enrichment of acid fast cells may be enforced by centrifuging the mixture at any suitable velocity, for about 10 sec, 20 sec, 30 sec, 45 sec, 1 min, 2 min, or longer. In a further, alternative embodiment, the enrichment of acid fast cells may be enforced by heating the mixture, e.g. up to a temperature of about 50 to 100°C.

The enriched acid-fast cells may comprise about 60 %, 70 %, 80 %, 85 %, or 90 % of the acid-fast cells in a sample used for the method of the present invention. Preferably, the enriched acid-fast cells may comprise about 91 %, 92 %, 93 %, 94 %, 95 %, 96 %, 97 %, 98 %, 99 % or 100 % of the acid-fast cells in a sample used for the method of the present invention. Of non-acid fast cells about 5 %, preferably less than about 5 %, e.g. about 4 %, 3 %, 2 %, 1%, 0.5 %, 0.1 %, 0.05 %, 0.001 % or 0 % may be present in the oil solution as described herein.

Concomitant to the enrichment of the cells in said oil solution, an accumulation of said dye as defined herein above in the interior of the acid-fast cell occurs. Typically, a dye molecule a defined herein may equilibrate easily with very hydrophobic or lipophilic cell wall portions or with the very hydrophobic or lipophilic cell wall of an acid-fast cell. Upon such an equilibration process, a dye molecule may move towards said cell wall or cell wall portions. In this process, a dye molecule may reach the inner part of the cell wall or cell wall portion and enter the interior of the acid-fast cell. Due to a different chemical environment of the interior of the acid-fast cell in comparison to its cells wall, the oil-solubility and hence the tendency to enter the cell wall of the dye molecule changes. Subsequently, the dye molecule is trapped in the interior of the acid-fast cell and cannot reenter the cell wall or cell wall portion of said cell. In specific embodiments, the change of chemical environments, e.g. pH or redox state, may also give rise to color changes of a dye molecule to be used within the context of the present invention.

The term "accumulation of the dye" as used herein refers to the concentration of about 75%, 80%, 90%, preferably of about 95 %, 96 %, 97 %, 98 % or more of the dye molecules present in the oil solution in the interior of the acid fast cell.

The "interior" of the acid fast cell is understood as all cell compartments or cell portions inside or beyond a very hydrophobic or lipophilic cell wall, preferably as all cell compartments or cell portions inside or beyond a mycolic acid comprising cell wall or cell wall portion, more preferably as all cell compartments or cell portions inside or beyond a alpha-, methoxy-, and keto-mycolic acid comprising cell wall or cell wall portion. In certain embodiments of the present invention, the interior of the acid-fast cell may be in a viable form. In alternative embodiments, the interior of the acid-fast cell may be in a non-viable or decontaminated state.

In a further embodiment of the present invention the method as described herein above additionally comprises as step (c) a molecular diagnostic step. The term "molecular diagnostic step" as used herein refers to all suitable diagnostic techniques or methodologies allowing to identify or detect an acid-fast cell, or to assess its molecular situation or genomic sequence, the assessment of the presence of different species in a sample, the pathogenicity of an acid-fast cell, the number of such cells etc. Corresponding suitable techniques such as polymerase chain reaction (PCR), ligase chain reaction, fragment sequencing, genomic sequencing, immunostaining, immuno-based identification techniques, antibody-based assays etc. would be known to the person skilled in the art and may be used as such or upon necessary adjustments after an initial enrichment and staining activity as described herein. For example, where possible, an adjustment to a performance of the technique in oil may be carried out. The performance of the technique(s) in aqueous environments is preferred.

Particularly preferred is the detection of an acid-fast cell via a molecular detection technique. Examples of such molecular detection techniques are the use of acid-fast cell, in particular acid-fast cells or Mycobacteria-specific antibodies, or fragments thereof, or modified versions thereof, e.g. antibodies modified with labels etc.

In a further preferred embodiment, a molecular diagnostic step as described herein may comprise the extraction of water soluble contents from lysed acid-fast cells. The term "extraction" as used herein refers to the separation of oil and water phases or of lipophilic and lipophobic portions of acid-fast bacteria. Such a separation may preferably be carried out with suitable buffers such as PBS, or a PCR-buffer, or may be performed within a lysis buffer. In order to be applicable to a separation technique, the acid-fast cells is preferably lysed, e.g. by the use of a lysis solution as described herein above.

Among water soluble contents to be separated are DNA, RNA, proteins and sugar components, preferably DNA, RNA and proteins. Such entities may be recovered or secured according to suitable techniques. For example, for the employment of RNA molecules, the use of RNA degrading enzyme or RNAse inhibitors is envisaged. Similarly, if DNA is to be analysed, DNA degrading enzyme or DNase inhibitors may be used. For the subsequent analysis of proteins, the use of protein degrading enzyme or protease inhibitors is envisaged. Also envisaged is the employment of a cocktail of different inhibitors in order to carry out several assays simultaneously or with the same cell material. Further suitable recovery or securing measures would be known to the skilled person or can be derived from suitable textbooks and are also envisaged by the present invention. In order to prepare the sample for subsequent molecular analysis steps, inhibitor molecules such as RNase, DNase or protease inhibitors may be added to the oil solution and/or the lysis solution at the beginning of the method as described herein. For the molecular diagnostic analysis of nucleic acids, e.g. RNA or DNA, the nucleic acids may be sheared or cut into smaller fragments (e.g., by mechanical shearing or restriction enzyme digestion). Preferred is a shearing process leading to nucleic acid molecules of a size of 50 to 500 nucleotides, preferably of 200 nucleotides. Typically, a shearing technique by focused ultrasound may be used, e.g. based on any suitable apparatus. Heterogeneous samples may further be purified to remove substantially all non-nucleic acid molecules prior to the molecular diagnostic analysis. Additionally, or alternatively, heterogeneous samples may be processed by separation techniques such as capillary electrophoresis, e.g. in order to deselect molecules outside of a desired length range, preferably molecules having a size outside of a range of 50 to 500 nucleotides.

The performance of a molecular detection technique as mentioned herein above may preferably be carried out in said aqueous solution, more preferably in said aqueous solution comprising inhibitor molecules as defined herein above.

A "sample" as used herein refers to any sample, which contains, comprises, essentially consists of, or consists of one or more acid-fast cells as defined herein. In a preferred embodiment of the present invention, said sample is a stool sample, whole blood sample, serum sample, plasma sample, tears sample, saliva sample, nasal fluid sample, sputum sample, ear fluid sample, genital fluid sample, breast fluid sample, milk sample, colostrum sample, placental fluid sample, amniotic fluid sample, perspirate sample, synovial fluid sample, ascites fluid sample, cerebrospinal fluid sample, bile sample, gastric fluid sample, aqueous humor sample, vitreous humor sample, gastrointestinal fluid sample, exudate sample, transudate sample, pleural fluid sample, pericardial fluid sample, semen sample, upper airway fluid sample, peritoneal fluid sample, a fluid sample harvested from a site of an immune response, a fluid sample harvested from a pooled collection site, bronchial lavage sample, or urine sample, a soil sample or environmental water sample, or an oil waste sample. In further embodiments, the sample may also be a sample containing, comprising, essentially consisting of, or consisting of biopsy material, e.g. of any suitable organ or tissue, e.g. the lung, the muscle, brain, liver, skin, pancreas, stomach, etc. In a particularly preferred embodiment the sample is sputum sample

In some embodiments, acid-fast cells may be directly obtained from samples. In other situations, samples may be subjected to sample preparation techniques, e.g. based on standard protocols, including, for example, complete or partial purification, which renders the acid-fast bacteria more accessible to the staining methodology described herein, separation or pre-separation of acid-fast cells from other cells, in particular from other bacteria, e.g. due to size difference, binding behavior, lysis sensitivity etc. Such separation steps may, for example, be centrifugation steps. Furthermore, samples may be homogenized with physiologically acceptable buffer and detergent, sputum samples may be liquefied and fractionated. In a particularly preferred embodiment of the present invention, a liquefaction may be carried out with the help of a lysis solution as defined herein. Furthermore, antibiotics or bactericides may be added to samples to prevent further growth of any organisms present. Whole cells may also be removed or may be lysed to release their contents.

In another aspect the present invention relates to the use of an oil solution comprising an oil-soluble dye and a lysis solution in which said dye is less soluble than in said oil solution for specifically staining an acid-fast cell. The oil solution, the oil-soluble dye and the lysis solution correspond to those described herein above and below. Said oil solution comprising oil-soluble dye may preferably be used such that first an oil solution comprising an oil-soluble dye is used for contacting an acid-fast cell derived from a sample with said oil solution. Said lysis solution in which said dye is less soluble than in said oil solution may subsequently be used for mixing it with the previously obtained solution comprising an acid-fast cell in said oil solution. After having carried out these activities a specifically stained acid-fast cell may be obtained.

In another aspect the present invention relates to a kit of parts for specifically staining an acid-fast cell. The kit comprises an oil solution comprising an oil-soluble dye as defined herein above or below, and a lysis solution in which said dye is less soluble than in said oil solution as defined herein above or below. The kit components may be provided in different vessels or entities, e.g. in different bottles, or in different locations of a device. The kit may further comprise a leaflet with information on its use, and/or solutions, buffers and/or ingredients for performing additional reactions, e.g. molecular diagnostic analyses as described herein. Such ingredients may comprise PCR primers, enzymes for molecular diagnosis, e.g. DNA amplification or analysis, nucleotides, ions, buffers etc.

In a further aspect the present invention relates to a sample collector suitable for processing and staining an acid-fast cell, comprising a cap element containing a lysis solution, wherein said cap element comprises a predetermined breaking point allowing the lysis solution to flow out of said cap element, and a receptacle containing an oil solution comprising an oil-soluble dye, wherein said dye is less soluble in said lysis solution than in said oil solution. The oil solution within the receptacle element may be provided in any suitable location, preferably at the bottom. In alternative embodiment, the receptacle element may be provided without the oil solution. In this embodiment, the oil solution comprising an oil-soluble dye may be added at a later point in time. It is also envisaged that the oil solution and the oil-soluble dye are not added at the same time, but one after the other, e.g. first the oil and subsequently the dye or vice versa.

The term "predetermined breaking point" as used herein refers to a point in the cap structure which is supposed to be broken once the cap is used to seal the sample collector. Upon its breaking the cap element allows a lysis solution as defined herein to flow out of said cap element. This structure advantageously allows to provide for a mixture of the sample, the oil solution comprising an oil-soluble dye and the lysis solution without the necessity of reopening the sample collector. Thereby the safety of medical personal and diagnostic assistants may be increased significantly.

The sample collector may further be equipped with handles or handle like structures allowing a patient to easier use it in order to provide a sample, e.g. a sputum sample. The sample collector may further be equipped with an airtight sealing functionality avoiding any escape of enclosed samples or acid-fast cells therein.

In further specific embodiments of the present invention, the sample collector may additionally have the possibility of carrying a label with a patient's name or address, hospital's name or address, physician's name or address, or any suitable identification number, or any other useful information. Such a label may be provided by hand, e.g. within a labeling field where the information can be filled in by hand, e.g. with the help of a pen etc. Alternatively, the sample collector may carry a barcode or matrix code whose information content may subsequently be linked to the patient information etc., or may have a predetermined area, where a barcode or matrix code may be placed or adhered. In further envisaged embodiments, the sample collector may be equipped with an electronic or computerized device or a chip, e.g. a RFID-chip, or any other suitable means allowing its electronic, computerized or remote identification and the corresponding identification of its content with a suitable reading and/or coding equipment or apparatus.

In further, alternative embodiments of the present invention the predetermined breaking point of the cap element may remain intact upon a closure of the sample collector and be broken subsequently without the necessity of reopening the sample collector, e.g. by a remote control mechanism. In this constellation it may be possible to cultivate or incubate the cells in the sample without lysis, e.g. during a certain period of time. Furthermore, in this constellation the mixture of sample ingredients, oil and dye may be stable for a longer period and may accordingly be kept in a refrigerator or cool environment until the staining analysis is carried out.

The sample collector or its components, e.g. the cap element, may be provided in or comprise, or essentially consist or consist of any suitable material, e.g. plastic, glass, metal or any combination thereof. The present invention also envisages a method for the production of a sample collector as described herein, or of a portion thereof. The method may preferably also include the filling of the sample collector and/or the cap element with the corresponding solutions, i.e. oil solution, and lysis solution.

The sample collector or parts of it may be reusable, e.g. after a washing or cleaning step. For example, the cap element, may be a part of the sample collector, which has to be replaced, whereas the receptacle portion may be reused after a thorough cleansing procedure.

In a further, particularly preferred embodiment, the sample collector is a sample collector as depicted in Fig. 6, e.g. in Fig. 6A with intact cap element, showing the functionality of the cap element in Fig. 6B, where the cap element is broken.

In another aspect the present invention relates to the use of a sample collector as defined herein for specifically staining an acid-fast cell. The sample collector may essentially be used as depicted in Fig. 6: first a patient may provide a sample which is introduced into the sample collector which already contains an oil solution and an oil-soluble dye as described herein, e.g. a sputum sample is introduced by spitting directly into the sample collector (see Fig. 6A); subsequently, by closing the cap element, the predetermined breaking point in the cap element may be broken and a lysis solution may flow out of the cap element into the receptacle section, joining the sample, the oil solution and the oil-soluble dye (see Fig. 6B); subsequently, an enrichment of the acid-fast cells after a mixture of the components may be carried out; this enrichment may be assisted by the application of external heat, e.g. of a heating up to a temperature of 50°C to 100°C (see Fig. 6C); alternatively the enrichment may be obtained after a few moments of resting the mixture in the sample collector of the sample collector, e.g. after several seconds, 30 sec, 45 sec, 1 min, 2 min, 2.5 min, 3 min, 4 min, 5 min or longer of resting the mixture in the sample collector, e.g. at room temperature; in a further alternative, the enrichment may be enforced by centrifuging the mixture, e.g. at a suitable velocity for about 10 sec, 20 sec, 30 sec, 45 sec, 1 min, 2 min, or longer subsequently, i.e. as result, a separation of the phases may have taken place, with acid-fast bacteria comprising the oil-soluble dye in the upper oily phase (see Fig. 6D); subsequently, enriched cells may be derived or obtained from the sample collector on a suitable object, e.g. a slide; the object, e.g. slide, may be used in microscopy directly or be introduced into a suitable detection device, e.g. a handheld instruments allowing for a visual analysis of staining results (see Fig. 6E and 6F).

In further particularly preferred embodiment of the present invention the acid-fast cell which is subject to a staining method or use of the present invention or which is to be stained with the help of a kit of parts or a sample collector as described herein is a mycobacterium. Among the preferred group of mycobacteria, particularly preferred is a bacterium of the Mycobacterium tuberculosis complex, or a bacterium of the Mycobacterium avium complex, or a bacterium of the group of mycolactone-producing mycobacteria, or a leprosy causing mycobacterium; or a bacterium of the genus Nocardia, e.g. Nocardia asteroids; or a bacterium of the genus Rhodococcus, e.g. Rhodococcus equi. In an even more preferred embodiment of the present invention the acid-fast cell which is subject to a staining method or use of the present invention or which is to be stained with the help of a kit of parts or a sample collector as described herein is Mycobacterium tuberculosis, Mycobacterium bovis, Mycobacterium africanum, Mycobacterium canetti, Mycobacterium microti, Mycobacterium avium, Mycobacterium ulcerans, Mycobacterium leprae, or Mycobacterium lepromatosis. Most typically, the method according to the present invention may be used for the staining of Mycobacterium tuberculosis. Details of the morphology, pathogenicity and molecular or histological behavior of the bacteria may be known to the person skilled in the art, or can be derived from suitable qualified literature, e.g. from Bergey's Manual of Systematic Bacteriology, 2nd ed., or Bergey's Manual of Determinative Bacteriology, 9th ed.

Any of the above mentioned bacteria may be specifically stained based on a method according to the invention or with the help of a kit of parts or a sample collector as described herein, and thus be distinguished from bacteria not belonging to the mentioned group. A differentiation between different members of the group of acid fast cells or mycobacteria by the current staining approach may accordingly only become possible on the basis of secondary features, which are not related to the staining, e.g. morphological differences, size differences, metabolistic differences, the presence of chemical ingredients, molecular or genetic differences etc. However, based on the origin of the sample, e.g. sputum sample, and given the possibility of carrying out additional molecular diagnostic tests, a further in-group differentiation may either not be required or can be obtained by secondary analysis approaches, e.g. molecularly, or by additional morphological or microbiological tests if required.

The following examples and figures are provided for illustrative purposes. It is thus understood that the examples and figures are not to be construed as limiting. The skilled person in the art will clearly be able to envisage further modifications of the principles laid out herein.

### EXAMPLES

### Example 1- Staining of mycobacteria with Acridine orange

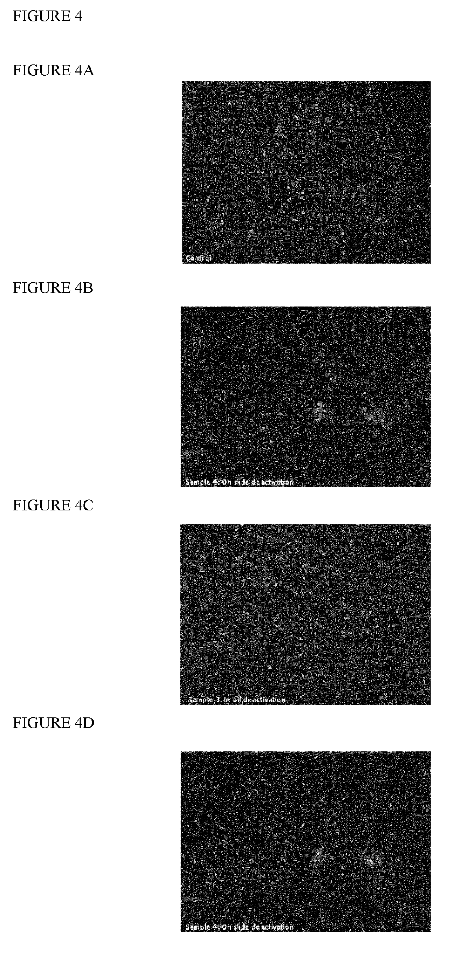

For the staining of mycobacteria with Acridine orange (see Fig. 4, 5 and 6) mycobacteria were directly dissolved in paraffin oil containing 100 µg/ml Acridine orange. Other molecules showing similar properties can also be used.

To dissolve Acridine orange in oil, Acridine orange was first dissolved in 2.5 ml chloroform (CHCl₃, 100 mg/ml), which was then mixed with 0.5 ml methanol, CH₃OH . This was subsequently mixed into the oil to reach the desired final concentration of dye. The solution was incubated at room temperature overnight to let the organic solvents (chloroform and methanol) evaporate.

### Example 2 — Staining of mycobacteria in oil after extraction from aqueous solution and inactivation at different steps

Mycobacteria were resuspended in water and divided over various tubes (50 µl each). To each tube 950 µl water were added (*, see Fig. 4B). 10 µl mineral oil containing 100 µg/ml Acridine orange were added and mixed well during 1 minute. After a short spin (10 seconds reaching a maximal speed of 100 x g) with a centrifuge the aqueous phase was removed and the oil harvested (**, see Fig. 4C). The oil was then transferred to a microscopy slide (***, see Fig. 4D). The four samples were treated identically, except that at the steps denoted with an * (*, **, and ***) the samples 2, 3 and 4, respectively, were heat inactivated by heating it up during 20 minutes to 80°C.

As can be derived from Fig. 4 the staining of mycobacteria in oil after extraction from aqueous solution works. Furthermore, heat inactivation of the bacteria at different steps does not affect the structural integrity of the mycobacteria. Staining is thus possible under these conditions. Only in Fig. 4B (i.e. inactivation in water) a shift in color can be seen. This can also be seen when using different concentrations of dye (not shown here).

### Example 3 — Staining of mycobacteria with different concentrations of Acridine orange

Mycobacteria were resuspended in oil containing varying concentrations of Acridine orange base. Lower concentrations of Acridine orange base (200 µg/ml) stain the mycobacteria green/yellow. High concentrations of Acridine orange base (1 mg/ml) color the background green while staining most bacteria red, making even low concentrations of mycobacteria easily distinguishable from background. The ratio of red versus green bacteria was estimated visually after taking microscopic pictures (see Fig. 5).

This allows for fine-tuning that can be used to distinguish bacteria more easily from background or artefacts.

## Claims

1. A method for specifically staining an acid-fast cell, comprising the steps of:
(a) contacting an acid-fast cell derived from a sample with an oil solution comprising an oil-soluble dye; and
(b) mixing the acid-fast cell of step (a) with a lysis solution in which said dye is less soluble than in said oil solution,
wherein said mixing causes an enrichment of said acid-fast cell in said oil solution and concomitantly an accumulation of said dye, in the interior of the acid-fast cell.

2. The method of claim 1, additionally comprising as step (c) a molecular diagnostic step, preferably a detection of an acid-fast cell via a molecular detection technique.

3. The method of claim 2, wherein said molecular diagnostic step comprises the extraction of water soluble contents from lysed acid-fast cells, preferably of DNA or proteins, into an aqueous solution, followed by the performance of a molecular detection technique in said aqueous solution.

4. The method of any one of claims 1 to 3, wherein said sample is a stool, whole blood, serum, plasma, tears, saliva, nasal fluid, sputum, ear fluid, genital fluid, breast fluid, milk, colostrum, placental fluid, amniotic fluid, perspirate, synovial fluid, ascites fluid, cerebrospinal fluid, bile, gastric fluid, aqueous humor, vitreous humor, gastrointestinal fluid, exudate, transudate, pleural fluid, pericardial fluid, semen, upper airway fluid, peritoneal fluid, fluid harvested from a site of an immune response, fluid harvested from a pooled collection site, bronchial lavage, or urine sample, a soil or environmental water sample, a oil waste sample, or a biopsy material sample.

5. Use of an oil solution comprising an oil-soluble dye and a lysis solution in which said dye is less soluble than in said oil solution for specifically staining an acid-fast cell.

6. A kit of parts for specifically staining an acid-fast cell, wherein said kit comprises an oil solution comprising an oil-soluble dye, and a lysis solution in which said dye is less soluble than in said oil solution.

7. A sample collector suitable for processing and staining an acid-fast cell, comprising a cap element containing a lysis solution, wherein said cap element comprises a predetermined breaking point allowing the lysis solution to flow out of said cap element, and a receptacle containing an oil solution comprising an oil-soluble dye, wherein said dye is less soluble in said lysis solution than in said oil solution.

8. The method of any one of claims 1 to 4, the use of claim 5, the kit of claim 6 or the sample collector of claim 7,
(i) wherein said lysis solution is an alkaline lysis solution having a pH of about 10.5 or higher, preferably of about 12 or higher, and wherein said dye is an alkaline dye; or
(ii) wherein said lysis solution is an acid lysis solution having a pH of about 4 or lower, preferably of about 3.5 or lower, and wherein said dye is an acid dye; or
(iii) wherein said lysis solution is a redox lysis solution comprising a lysis solution with a lower oxidative power than the cytoplasm inside an acid fast cell and wherein said dye is a basic redox dye which is in an uncharged, oil soluble state.

9. The method, use, kit or sample collector of claim 8, wherein said alkaline dye is Acridine orange, 1,4-phenylenediamine, Bismarck brown, quinacrine, N-4-amino-2,5 diethoxy phenyl benzamide, Nigrosin, Nile Blue A, Sudan Blue II, Sudan Black B or Solvent Green 3, preferably Acridine orange.

10. The method, use, kit or sample collector of claim 8, wherein said acid dye is 2-naphthol, 4-chloro-1-naphthol, oil red o, rose Bengal, bromophenol blue, carminic acid, phloxine b, picric acid, eosin (solvent red 45), erythrosine, fluoresceine 5 (6) isothiocyanate, hematoxyline, hesperetin, morin, Resorufin sodium salt, Sudan IV or Sudan Orange G.

11. The method, use, kit or sample collector of claim 8, wherein said basic redox dye is Auramine, azure a, azure b, azure II, brilliant green, brilliant cresyl blue, fuchsin, ethyl violet, toluidine blue, anus green B, cresyl violet, methylene blue, methylene green, malachite green, methyl violet, New Methylene Blue, Neutral Red, Phenosafranine, Pinacyanol iodide, Rhodamine B, or Safranin O.

12. The method of any one of claims 1 to 4, or 8 to 11, the use of any one of claims 5 or 8 to 11, the kit of any one of claims 6 or 8 to 11, or the sample collector of any one of claims 7 to 11, wherein said oil has a partition coefficient of more than 10⁴ in octanol/water, preferably a partition coefficient of between 10⁵ and 10⁸ in octanol/water.

13. The method, use, kit or sample collector of claim 12, wherein said oil is an oil from an animal source, such as fish oil, lard oil, cod liver, or butter; an oil from a vegetable source, such as olive oil, sunflower oil, peanut oil, soybean oil, or corn oil; a mineral oil, such as paraffin oil, naphtenic oil, or aromatic oil; or a silicon oil.

14. The method of any one of claims 1 to 4 or 8 to 13, the use of any one of claims 5 or 8 to 13, the kit of any one of claims 6 or 8 to 13, or the sample collector of any one of claims 7 to 13, wherein said acid-fast cell is a mycobacterium, preferably a bacterium of the Mycobacterium tuberculosis complex, a bacterium of the Mycobacterium avium complex, a bacterium of the group of mycolactone-producing mycobacteria, or a leprosy causing mycobacterium; or a bacterium of the genus Nocardia, preferably Nocardia asteroids; or a bacterium of the genus Rhodococcus, preferably Rhodococcus equi.

15. The method, use, kit or sample collector of claim 14, wherein said mycobacterium is Mycobacterium tuberculosis, Mycobacterium bovis, Mycobacterium africanum, Mycobacterium canetti, Mycobacterium microti, Mycobacterium avium, Mycobacterium ulcerans, Mycobacterium leprae, or Mycobacterium lepromatosis, preferably Mycobacterium tuberculosis.
